# EUROPÄISCHE PATENTSCHRIFT

(11) **EP 2 682 603 B1**
(45) Veröffentlichungstag und Bekanntmachung des Hinweises auf die Patenterteilung: **08.11.2017**
(21) Anmeldenummer: 13174610.9
(22) Anmeldetag: 02.07.2013
(51) Int. Cl.: F04B 43/12

(54) **Schlauchrollenpumpe mit schwenkbarem Deckel und medizinisches Gerät zur extrakorporalen Blutbehandlung mit Schlauchrollenpumpe**
Hose reel pump with pivotable cover and medical device for extracorporeal blood treatment with hose reel pump
Pompe à rouleau tubulaire avec couvercle pivotant et appareil médical pour le traitement extracorporel du sang doté d'une pompe à rouleau tubulaire

(30) Priorität: 03.07.2012 DE 102012105919
(43) Veröffentlichungstag der Anmeldung: 08.01.2014
(73) Patentinhaber: B. Braun Avitum AG, 34212 Melsungen (DE)
(72) Erfinder: Schäfer, Oliver, 36286 Neuenstein (DE)
(74) Vertreter: Winter, Brandl, Fürniss, Hübner, Röss, Kaiser, Polte - Partnerschaft mbB

(56) Entgegenhaltungen:
- WO-A1-2011/107326
- DE-T2- 3 872 053
- DE-T2- 69 615 633
- US-A- 6 077 246
- US-B1- 6 203 528

## Beschreibung

Die Erfindung betrifft eine Schlauchrollenpumpe für ein medizinisches Gerät zur extrakorporalen Blutbehandlung, umfassend ein Pumpengehäuse, das eine gebogene Lauffläche und einen innerhalb der Lauffläche drehbaren Rotor aufweist, wobei ein Schlauchsegment schlaufenförmig zwischen die Lauffläche und den Rotor einbringbar ist und am Pumpengehäuse Mittel zur Anbringung des Schlauchsegments vorgesehen sind, mit denen wenigstens ein Ende des Schlauchsegments um eine Drehachse schwenkbar am Pumpengehäuse anbringbar ist. Dabei ist das Verschwenken des Schlauchsegments durch Verschwenken eines Kippelements durchführbar, an dem das Schlauchsegment wenigstens teilweise angebracht ist.

Die Erfindung betrifft ferner ein medizinisches Gerät zur extrakorporalen Blutbehandlung mit einer solchen Schlauchrollenpumpe.

In medizinischen Geräten zur extrakorporalen Blutbehandlung (Dialyse) werden oftmals Schlauchrollenpumpen eingesetzt, welche das entnommene Blut des Patienten zu einem Dialysator und zum Patienten zurückfördern. Solche Schlauchrollenpumpen arbeiten peristaltisch, wobei ein schlaufenförmiges Schlauchsegment an einer entsprechend gebogenen Lauffläche des Pumpengehäuses anliegt. Ein innerhalb der Lauffläche liegender Rotor der Pumpe bewegt sich dann mit seinen Außenkanten entlang des Schlauchsegments, wobei es den Schlauch lokal eindrückt und so mit den elastischen Materialeigenschaften des Schlauchsegments eine Blutförderung durch das Schlauchsegment ermöglicht. Dafür wird das Blut dem Schlauchsegment über einen ersten Anschluss zugeführt und über einen weiteren Anschluss am anderen Ende des Schlauchsegments wieder abgeführt.

Das Schlauchsegment bildet so beispielsweise zusammen mit den zu- und abführenden Leitungen und mehreren Luftfängern ein sogenanntes Überleitsystem, mit dem Blut des Patienten zu einem Dialysator und zum Patienten zurückgefördert wird. Derartige Überleitsysteme werden vorzugsweise nach jeder Behandlung ausgetauscht und nicht wieder für andere Patienten verwendet. Ein benutztes Schlauchsegment muss somit aus der Pumpe entfernt werden, bevor ein neues Überleitsystem in das Gerät eingebracht wird. Um die Handhabung während dieses Ablegens und Aufrüstens des Überleitsystems zu erleichtern, ist es ferner bekannt, an beiden Enden des Schlauchsegments jeweils einen Konnektor vorzusehen, der mit einer zu- bzw. abführenden Leitung verbunden werden kann.

Zur Aufnahme eines Schlauchs innerhalb einer Rollenpumpe beschreibt beispielsweise das Patent US 8,047,819 B2 Haltevorrichtungen, die lösbar am Pumpengehäuse angebracht sind. So können für verschiedene Schlauchgrößen und -arten unterschiedliche Haltevorrichtungen an der Pumpe montiert werden. Eine Haltevorrichtung weist dabei eine Klemmvorrichtung mit wenigstens einer schwenkbaren Klemmbacke auf, die eine halbkreisförmige Ausnehmung aufweist, so dass ein Schlauch in dieser halbkreisförmigen Ausnehmung und einer gegenüber liegenden, ebenfalls halbkreisförmigen Ausnehmung einer anderen Klemmbacke gehalten werden kann. Die Klemmbacken können auch mehrere dieser Ausnehmungen aufweisen, so dass mehrere Schläuche gleichzeitig aufgenommen werden können.

Weitere Haltevorrichtungen für einen Schlauch innerhalb einer Schlauchrollenpumpe sind beispielsweise in der DE 38 72 053 T2 oder der US 6 203 528 B1 beschrieben. Auch diese Haltevorrichtungen sind lösbar am Pumpengehäuse angebracht.

Des Weiteren ist beispielsweise aus der US 6 077 246 A ein verschwenkbarer Deckel für eine Pumpe eines medizinischen Geräts bekannt, wobei es sich bei der Pumpe nicht um eine Schlauchrollenpumpe handelt.

Darüber hinaus sind beispielsweise aus der WO 2011/107326 A1 oder der DE 696 15 633 T2 automatische Systeme bekannt, die das Ein- und Ausfädeln des Schlauchs in die Pumpe übernehmen und somit erleichtern sollen. Oftmals ist dabei zum Ausfädeln ein Aktuator zu betätigen, der das System beispielsweise über einen Linearantrieb aus seiner Therapieposition in eine Ausfädelposition bewegt. Hierzu kann es bei solchen Systemen erforderlich sein, einen Schalter/Knopf an dem medizinischen Gerät zu bedienen bzw. einen Softwarebutton auf einer Benutzeroberfläche zu berühren.

Ferner sind Multikonnektoren bekannt, die beide Anschlüsse für zu- bzw. abführende Leitungen in einem Bauteil vereinigen, das dann in eine Aufnahme des Pumpengehäuses eingebracht werden kann. Über die geometrische Form derartiger Multikonnektoren kann auch ihre Präsenz in der Pumpe detektiert werden, indem beispielsweise ein zylindrischer Abschnitt des Multikonnektors während des Einsetzvorgangs einen Stößel betätigt, dessen axiale Position über eine Lichtschranke abgefragt wird. Gleichzeitig ist dieser Stößel Bestandteil eines im Pumpengehäuse montierten elektromechanischen Aktuators, der den Multikonnektor über einen Linearantrieb auswerfen kann.

Um ein Schlauchsegment an einer Schlauchrollenpumpe jeweils in die Ein- und Ausfädelposition zu bewegen, sind darüber hinaus aus der JP 2008-000425 mehrere Varianten eines Schwenkbauteils bzw. Kipphebels bekannt, an dem beide Enden des schlaufenförmigen Schlauchsegments angebracht werden können. In einer ersten Schwenkposition des Kipphebels befindet sich das Schlauchsegment dann in einer Lage, in welcher der Einfädelvorgang gestartet werden kann, während das Schlauchsegment in einer anderen Schwenklage ausgefädelt werden kann. Der automatische Ein- und Ausfädelvorgang erfolgt durch Führungsstifte am Umfang des Rotors, welche den Schlauch bei Drehung des Rotors in das Pumpengehäuse hinein- oder aus ihm herausdrücken.

Während der Therapie und vorzugsweise auch bei automatisierten Einfädel- und Ausfädelvorgängen wird die Pumpe üblicherweise mit einem schwenkbaren Deckel verdeckt, der manuell von dem Bediener geschlossen bzw. geöffnet werden kann. Der Deckel bildet so einen Eingreifschutz, um während der motorgetriebenen Drehung des Rotors Eingriffe in die Pumpe zu verhindern, die zu Verletzungen und Schäden an der Pumpe führen könnten.

Ausgehend hiervon ist es die Aufgabe der Erfindung, eine Schlauchrollenpumpe für ein medizinisches Gerät zur extrakorporalen Blutbehandlung mit einem Deckel bereitzustellen, der zusätzlich zu der Funktion eines Eingreifschutzes auch die Anbringung eines Schlauchsegments und automatisierte Ein- und Ausfädelvorgänge unterstützt.

Aufgabe der Erfindung ist es ferner, ein medizinisches Gerät zur extrakorporalen Blutbehandlung mit einer solchen Schlauchrollenpumpe bereitzustellen.

Erfindungsgemäß wird diese Aufgabe durch eine Schlauchrollenpumpe gemäß dem unabhängigen Anspruch 1 gelöst. Vorteilhafte Weiterbildungen der Schlauchrollenpumpe ergeben sich aus den Unteransprüchen 2-15. Die Aufgabe wird ferner durch ein medizinisches Gerät zur extrakorporalen Blutbehandlung gemäß Anspruch 16 gelöst.

Die erfindungsgemäße Schlauchrollenpumpe eignet sich für ein medizinisches Gerät zur extrakorporalen Blutbehandlung und umfasst ein Pumpengehäuse, das eine gebogene Lauffläche und einen innerhalb der Lauffläche drehbaren Rotor aufweist, wobei ein Schlauchsegment schlaufenförmig zwischen die Lauffläche und den Rotor einbringbar ist und am Pumpengehäuse Mittel zur lösbaren Anbringung bzw. zum Halten des Schlauchsegments vorgesehen sind, mit denen wenigstens ein Abschnitt bzw. ein Endabschnitt des Schlauchsegments um eine Drehachse bezüglich des Pumpengehäuses geschwenkt werden kann. Dabei ist das Verschwenken des Schlauchsegments durch Verschwenken eines Kippelements durchführbar, an dem das Schlauchsegment wenigstens teilweise angebracht ist bzw. das einen Teil des Schlauchsegments hält.

Erfindungsgemäß weist die Schlauchrollenpumpe einen Deckel auf, der schwenkbar am Pumpengehäuse gelagert ist und mit dem das Pumpengehäuse wenigstens teilweise abdeckbar ist, wobei der Deckel so ausgeformt und an dem Pumpengehäuse angebracht ist, dass er im geschlossenen Zustand bei Verschwenken des Schlauchsegments in Richtung des geschlossenen Deckels ein Gegenlager für das Kippelement bildet, durch welches das daran angebrachte Schlauchsegment in einer definierten Lage gegenüber dem Pumpengehäuse ausrichtbar ist. Ferner weist die Schlauchrollenpumpe wenigstens Mittel zum automatisierten Ausfädeln des Schlauchsegments aus der Lauffläche in dieser definierten Lage auf.

Neben der reinen Funktion als Eingreifschutz weist die erfindungsgemäße Pumpe somit einen Deckel auf, der auch die Anbringung des Schlauchsegments und wenigstens den automatisierten Ausfädelvorgang unterstützt, da er im geschlossenen Zustand ein Gegenlager bildet, gegen den ein Kippelement gezogen werden kann, welches das Schlauchsegment in eine definierte Ausfädelposition bringt. Dabei dient der Deckel als Anschlag und bietet ferner Raum, um das Kippelement verschwenken zu können.

Der Deckel erlaubt im geschlossenen Zustand ein Verschwenken des Kippelements und eines darin gehaltenen Teils des Schlauchsegments. Der Deckel kann so gestaltet sein, dass der Deckel im geschlossenen Zustand einen Schwenkraum definiert, der im Wesentlichen nur eine bestimmte und gewünschte Schwenkbewegung des Kippelements zwischen einer Einfädelposition und einer Ausfädelposition des Schlauchsegments zulässt.

Dabei kann das Kippelement auf verschiedene Arten ausgeführt sein. Beispielsweise kann das Kippelement fester Bestandteil der Schlauchrollenpumpe sein und Mittel zur lösbaren Anbringung wenigstens eines Endes des Schlauchsegments aufweisen. Beide Enden des Schlauchsegments können dann temporär am Kippelement angebracht werden und durch Schwenken des Kippelements um eine Drehachse in ihrer Lage verändert werden. Vorzugsweise weist das Kippelement jedoch Mittel zur lösbaren Anbringung eines Endes des Schlauchsegments auf, während am Pumpengehäuse Mittel zur lösbaren Anbringung des anderen Endes des Schlauchsegments vorgesehen sind. Das erste Ende des Schlauchsegments steht somit am Pumpengehäuse fest, während das andere Ende durch Schwenken des Kippelements bewegt werden kann.

Der Vorteil der feststehenden ersten Aufnahme hat dabei insbesondere den Vorteil, dass diese Aufnahme nicht angehoben oder abgesenkt wird, sondern so an dem Pumpengehäuse fixiert ist, dass es in diesem Bereich zu keinen Einquetschungen des Schlauchs kommt. Hierdurch wird das Risiko eine Hämolyse während der Therapie verringert, so dass es insbesondere beim Einfädelvorgang vor der Therapie wichtig ist, dass der Schlauch nicht ungünstig verformt wird. Daher handelt es sich bei der feststehenden ersten Aufnahme vorzugsweise um diejenige Aufnahme, in deren Richtung der Schlauch beim Einfädeln durch den wenigstens einen Führungsstift leicht geschoben wird.

In einer anderen Ausführungsform des Kippelements ist dieses lösbar und schwenkbar an dem Pumpengehäuse anbringbar, indem es in eine Aufnahme am Pumpengehäuse einbringbar ist, welche ein Drehlager für das Kippelement bildet, wobei das Drehlager im Bereich eines Endes des Schlauchsegments angeordnet ist. Das Kippelement stellt in dieser Ausführungsform ein separates Bauteil dar, das lediglich temporär an dem Pumpengehäuse angebracht wird. Dabei können das Schlauchsegment und die zu- und abführenden Leitungen fest mit diesem Kippelement verbunden sein, das dann die Funktion eines Multikonnektors zwischen dem Schlauchsegment und den zu- und abführenden Leitungen übernimmt. Das Kippelement kann jedoch auch als wiederverwendbarer Adapter ausgeführt sein, an welchem ein Standard-Schlauchsystem lediglich für die Therapie angebracht wird. Dazu weist der Adapter entsprechende Aufnahmen auf.

Die Drehachse des Deckels und die Schwenkachse des Kippelements können senkrecht zueinander verlaufen.

Der Deckel der Pumpe kann an seiner Innenseite, welche im geschlossenen Zustand dem jeweiligen Kippelement zugewandt ist, eine Druckfläche aufweisen, die im Bereich des Drehlagers an dem Kippelement und/oder dem Schlauchsegment anliegt. So kann der Deckel dazu genutzt werden, einen Konnektor und/oder einen Teil des Schlauchsegments im Bereich der Drehachse des Kippelements in Position zu halten, wenn das Kippelement vom Pumpengehäuse weggeschwenkt wird. Das Kippelement bzw. das Schlauchsegment können sich somit nicht aus der entsprechenden Aufnahme lösen, da der Deckel gegen sie drückt, so dass der Deckel hierdurch neben der Funktion als Eingreifschutz auch eine Sicherungsfunktion hat.

Vorzugsweise ist das Kippelement um eine Drehachse schwenkbar am Pumpengehäuse angebracht, die quer zur Drehachse des Deckels am Pumpengehäuse verläuft. Beispielsweise verläuft die Drehachse des Deckels vertikal, während die Drehachse des Kippelements horizontal verläuft. Der Deckel kann dann einfach zur Seite geschwenkt werden, während der Einsetz- und Verschwenkvorgang des Kippelements durch ergonomisch günstige Handbewegungen durchgeführt werden kann.

Um das Kippelement manuell durch einen Bediener bewegen zu können, ist es bei geschlossenem Deckel wenigstens teilweise von außen zugänglich. Dabei ist es bei geschlossenem Deckel vorzugsweise von einer Seite zugänglich ist, die gegenüber der Drehachse des Deckels liegt.

Darüber hinaus ist es vorteilhaft, wenn die Geometrie des Kippelements eine Grifffläche ausbildet, mit welcher das Kippelement gegen den geschlossenen Deckel ziehbar ist. Vorzugsweise bildet die Geometrie des Deckels dann entsprechend an seiner Außenseite, welche im geschlossenen Zustand von dem Kippelement weg weist, eine Grifffläche aus, die bei Kontakt des Kippelements mit dem Deckel als Gegenlager in einem Abstand zu der Grifffläche des Kippelements steht, der größer als 3mm ist. Der Bediener der Pumpe muss beim Ziehen des Kippelements gegen den Deckel somit seine Finger nicht näher als 3mm zueinander bringen, da dies ansonsten unbequem und ergonomisch ungünstig wäre. Vorzugsweise liegt dieser Abstand daher eher in der Größenordnung von 5mm bis 20 mm.

Darüber hinaus können die Geometrie des Kippelements und die Geometrie des Deckels so aufeinander abgestimmt sein, dass das Kippelement nach dem Verschwenken gegen den geschlossenen Deckel als Gegenlager flächig an einer Anlagefläche des Deckels anliegt. So wird durch den Deckel ein Anschlag ausgebildet, der vom Bediener sicher als Endanschlag erkannt werden kann.

In einem Ausführungsbeispiel der Erfindung ist ferner vorgesehen, dass der Deckel bereichsweise eine hohle Auswölbung in eine Richtung aufweist, die im geschlossenen Zustand des Deckels von dem Pumpengehäuse weg weist. In dieser hohlen Auswölbung kann das Schlauchsegment vor dem automatisierten Einfädeln oder nach dem automatisierten Ausfädeln zum Liegen kommen, nachdem der Deckel bereits geschlossen wurde. Der Deckel kann somit geschlossen werden, ohne dass das Schlauchsegment zwischen Rotor und Deckel eingequetscht wird, oder die Höhe des Pumpengehäuses erheblich erhöht werden muss, um ausreichend Raum für das Schlauchsegment bereitzustellen.

Die Seitenkontur der hohlen Auswölbung folgt dabei im geschlossenen Zustand des Deckels im Wesentlichen der Kontur der Lauffläche, da in diesem Bereich zusätzlicher Raum für das Schlauchsegment benötigt wird, insbesondere nachdem es ausgefädelt wurde. Die hohle Auswölbung kann dabei jedoch im geschlossenen Zustand des Deckels in Richtung des Kippelements abflachen.

Von der Erfindung umfasst ist ferner ein medizinisches Gerät zur extrakorporalen Blutbehandlung, umfassend wenigstens eine Schlauchrollenpumpe mit einem Pumpengehäuse, das eine gebogene Lauffläche und einen innerhalb der Lauffläche drehenden Rotor aufweist, wobei ein Schlauchsegment eines extrakorporalen Blutkreislaufs zwischen die Lauffläche und den Rotor einbringbar ist, und dem Schlauchsegment Blut über eine zuführende Leitung zuführbar ist, während Blut von dem Schlauchsegment über eine abführende Leitung abführbar ist. Erfindungsgemäß umfasst das Gerät eine Schlauchrollenpumpe gemäß einer der beschriebenen Ausführungsformen.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines medizinischen Geräts zur extrakorporalen Blutbehandlung mit einer Blutpumpe;
- Fig. 2: eine schematische Aufsicht auf eine Schlauchrollenpumpe mit eingelegtem Schlauchstück und einem schwenkbaren Multikonnektor;
- Fig. 3a: eine schematische Seitenansicht einer Schlauchrollenpumpe mit geschlossenem Deckel und einem Multikonnektor gemäß Fig. 2 in der Einfädelposition;
- Fig. 3b: eine schematische Seitenansicht einer Schlauchrollenpumpe mit geschlossenem Deckel und einem Multikonnektor gemäß Fig. 2 in der Ausfädelposition;
- Fig. 4: eine schematische Aufsicht auf eine Schlauchrollenpumpe mit eingelegtem Schlauchstück und einem schwenkbaren Kipphebel;
- Fig. 5a: eine schematische Seitenansicht einer Schlauchrollenpumpe mit geschlossenem Deckel und einem Kipphebel gemäß Fig. 4 in der Einfädelposition;
- Fig. 5b: eine schematische Seitenansicht einer Schlauchrollenpumpe mit geschlossenem Deckel und einem Kipphebel gemäß Fig. 4 in der Ausfädelposition;
- Fig. 6: einen schematischen Querschnitt durch eine Schlauchrollenpumpe mit geöffnetem Deckel;
- Fig. 7a: einen schematischen Querschnitt gemäß Fig. 6 vor dem Einfädel- oder nach dem Ausfädelvorgang;
- Fig. 7b: einen schematischen Querschnitt gemäß Fig. 6 während der Therapie; und
- Fig. 8: eine dreidimensionale Ansicht des Deckels.

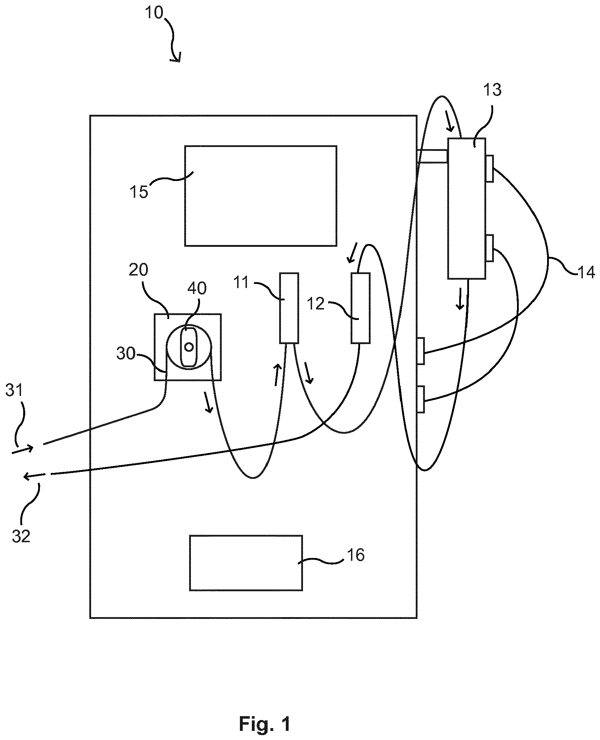

Fig. 1 zeigt eine schematische Darstellung der wesentlichen Grundkomponenten eines medizinischen Geräts 10 zur extrakorporalen Blutbehandlung mit einer Blutpumpe, wobei es sich bei der Blutpumpe um eine Schlauchrollenpumpe handelt. Die Schlauchrollenpumpe weist dabei ein Pumpengehäuse 20 auf, das typischerweise an der Frontseite des Dialysegeräts 10 angebracht ist.

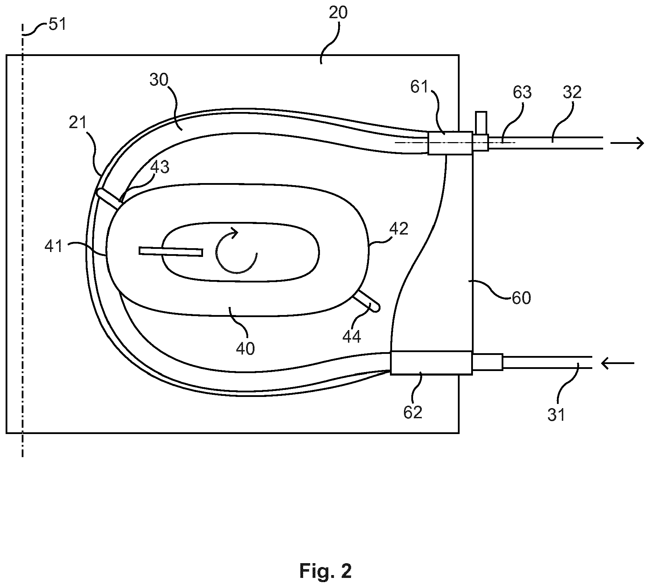

Dieser Schlauchrollenpumpe wird arterielles Blut 31 eines Patienten zugeführt und durch den extrakorporalen Blutkreislauf gefördert. Anschließend wird das Blut als venöses Blut 32 wieder zum Patienten zurückgeführt. Dabei wird das Blut mittels der Pumpe durch ein Überleitsystem gefördert, das an mehrere Komponenten des Dialysegeräts 10 angeschlossen ist, wobei ein Schlauchsegment 30 des Überleitsystems in die Blutpumpe eingelegt ist und ein Rotor 40 das Blut peristaltisch durch dieses Schlauchsegment 30 fördert, wie es einer vergrößerten Ansicht der Fig. 2 zu entnehmen ist.

Nach Durchlaufen der Blutpumpe gelangt das Blut zum Dialysator 13, nachdem es vorzugsweise zuvor einen arteriellen Luftfänger 11 durchlaufen hat. Im Dialysator 13wird das Blut durch Stoffaustausch mit einem Dialysat 14 gereinigt, welches dem Dialysator 13 zu- und abgeführt wird. Nach Durchlaufen des Dialysators 13 gelangt das Blut zu einem venösen Luftfänger 12 und wird anschließend dem Patienten zugeführt. Dieser Kreislauf des Bluts des Patienten ist in Fig. 1 durch Pfeile gekennzeichnet.

Die Einstellung von Parametern der Dialyse und die Überwachung der Therapie können über eine Anzeige-/Eingabeeinheit 15 erfolgen, die vorzugsweise als Touchscreen ausgebildet ist. Ferner weist das Dialysegerät 10 eine Steuer-/Regeleinheit 16 auf.

Fig. 2 stellt eine schematische Darstellung einer Schlauchrollenpumpe mit eingelegtem Schlauchsegment 30 und einer ersten Ausführungsform eines Kippelements dar, das als Multikonnektor 60 ausgeformt ist, an den sowohl das Schlauchsegment 30, als auch die zu- und abführenden Leitungen 31 und 32 des extrakorporalen Blutkreislaufs angeschlossen sind. Die Schlauchrollenpumpe weist dabei das Pumpengehäuse 20 auf, das für den Bediener des Geräts leicht zugänglich ist, wobei das Pumpengehäuse 20 mit einem (in Fig. 2 nicht dargestellten) Deckel 50 abdeckbar ist, der über eine Schwenkachse 51 vorzugsweise nach links schwenkbar ist, um Zugriff auf die Pumpe zu erhalten.

In dem Pumpengehäuse 20 ist durch eine Vertiefung im Gehäuse eine kurvenförmige Lauffläche 21 ausgebildet, in welche das Schlauchsegment 30 schlaufenförmig eingelegt werden kann, so dass seine beiden Schlauchenden aus dem Gehäuse 20 herausragen. Dabei kann die Vertiefung mit einer Seitenfläche in dem Pumpengehäuse 20 ausgebildet sein, die im Wesentlichen gleichmäßig senkrecht zur Frontseite des Geräts verläuft, oder die Lauffläche 21 ist ungleichmäßig durch eine Seitenfläche der Vertiefung ausgeformt, die konkav oder sogar in sich verdreht ausgeformt ist.

Innerhalb der Lauffläche 21 ist ein Rotor 40 angebracht, der einen elliptischen Umfang hat, so dass er das Schlauchsegment 30 bei der Rotation an seinen Hauptscheiteln 41, 42 bzw. daran angebrachten Rollen (nicht dargestellt) leicht zusammendrücken kann. Durch die Drehung des Rotors 40 im Uhrzeigersinn bewegt sich der Bereich eines zusammengedrückten Schlauchsegments ebenfalls im Uhrzeigersinn, bis sich der zugehörige Hauptscheitel wieder vom Schlauchsegment löst. In der Zeit hat der gegenüber liegende Hauptscheitel jedoch bereits wieder Kontakt zu dem Schlauchsegment 30 aufgenommen, so dass Blut jeweils in dem Bereich des Schlauchsegments 30, in dem es von dem Rotor 40 zusammendrückt wird, peristaltisch vom Pumpeneingang zum Pumpenausgang gefördert wird.

An den beiden Enden des Schlauchsegments 30 ist der Multikonnektor 60 angebracht, welcher eine Verbindung zu einer zuführenden Leitung 31 und einer abführenden Leitung 32 herstellt, durch welche somit Blut zu- und abgeführt wird. Diese Leitungen 31, 32 sind Teil des extrakorporalen Blutkreislaufs und sind mit verschiedenen Komponenten wie Luftfängern und dem Dialysator verbunden. Dabei ist der Multikonnektor 60 ein separates Bauteil, das fester Bestandteil des Einmalartikels sein kann, so dass der Multikonnektor 60 zusammen mit dem Schlauchsystem schwenkbar an der Pumpe montierbar ist. In einer alternativen Ausführungsform des Multikonnektors ist dieser als wiederverwendbarer Adapter ausgeformt, an dem ein Standard-Schlauchsystem temporär angebracht werden kann, um es an der Pumpe montieren zu können.

Der Multikonnektor 60 umfasst einen Grundkörper, der zwei gegenüber liegende Konnektorelemente 61 und 62 miteinander verbindet, die auch als Konnektoren bezeichnet werden können. Wenigstens das Konnektorelement 61 ist zylinderförmig ausgeführt, oder weist an seiner Außenfläche zumindest einen zylindrischen Bereich auf. Hierdurch kann dieses Konnektorelement 61 in eine entsprechend ausgeformte Aufnahme im Pumpengehäuse 20 eingebracht werden, wodurch sich ein Drehlager mit einer Drehachse 63 ergibt, um welche der Multikonnektor 60 schwenkbar ist. Das zweite Konnektorelement 62 ist vorzugsweise ebenfalls zylindrisch ausgeformt, wobei es sich auch um einen Zylinder mit abgestuften Außenflächen handeln kann. Die Zylinderachsen der beiden Konnektorelemente 61, 62 verlaufen dabei parallel zueinander, so dass die beiden Leitungen 31, 32 ebenfalls parallel in die Pumpe bzw. aus der Pumpe geführt werden.

Der Multikonnektor 60 ist somit in Fig. 2 um die Drehachse am Konnektorelement 61 aus der Zeichenebene heraus schwenkbar. In der eingeschwenkten Stellung sind Mittel vorgesehen, mit denen der Multikonnektor 60 am Pumpengehäuse 20 verrastbar ist. Beispielsweise ist das zweite Konnektorelement 62 dazu so ausgeformt, dass es in eine Aufnahme im Pumpengehäuse 20 einrastet, wenn es gegen das Pumpengehäuse 20 gedrückt wird. Es kann aber durch leichtes Ziehen auch wieder aus dieser Aufnahme gelöst werden, wenn der Multikonnektor 10 aus der Zeichenebene der Fig. 2 herausgeschwenkt wird. Der Grundkörper des Multikonnektors 60 ist daher vorzugsweise leicht elastisch ausgebildet, so dass er ein Einrasten in einer Aufnahme ermöglicht. Er kann jedoch auch relativ steif ausgeführt sein, wobei dann die Aufnahme im Pumpengehäuse 20 elastisch ausgeführt wäre.

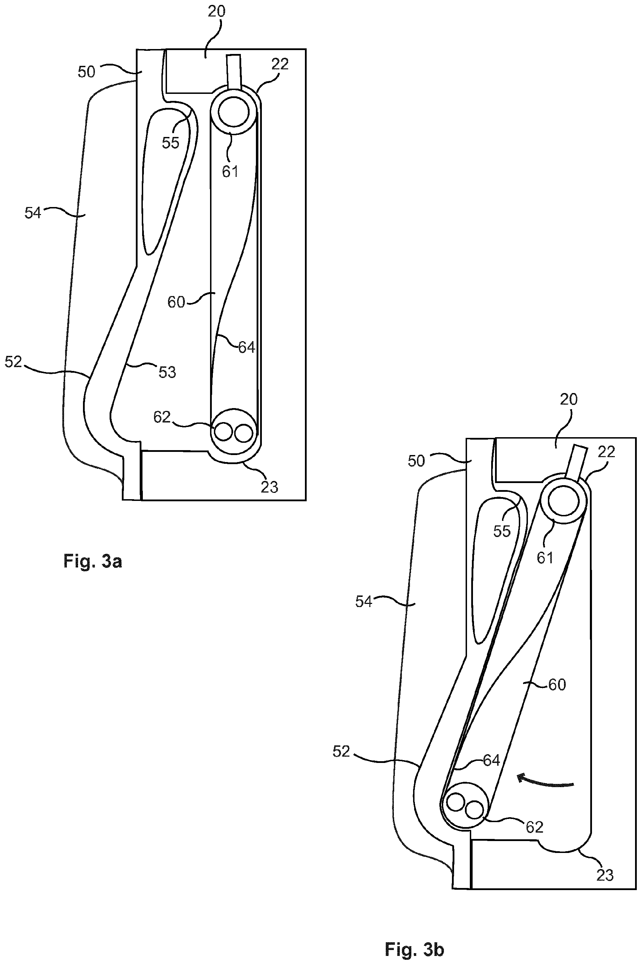

Die Flächen des Multikonnektors 60 können geometrische Codierungen aufweisen, um den Multikonnektor 60 an der Pumpe in die richtige Lage zu bringen. Ferner können Flächen als Griffflächen ausgebildet sein, mit denen der Multikonnektor 60 möglichst einfach und ergonomisch gegriffen und montiert werden kann. Um den Vorgang des Schwenkens um die Drehachse 63 näher zu erläutern, zeigen die Figuren 3a und 3b jeweils eine schematische Seitenansicht der Schlauchrollenpumpe mit montiertem Multikonnektor 60 und einem Deckel 50.

Dabei zeigt Fig. 3a einen eingelegten Multikonnektor 60, dessen oberes Konnektorelement 61 in eine halbkreisförmige Aufnahme 22 im Pumpengehäuse 20 eingebracht ist. Durch die zylindrische Form des Konnektorelements 61 und der Aufnahme 22 ergibt sich an dieser Stelle ein Drehpunkt, um den der Multikonnektor 60 schwenkbar ist. Dabei ist das untere Konnektorelement 62 ebenfalls in eine Aufnahme eingerastet. Dies kann beispielsweise durch eine halbkreisförmige Aufnahme 23 erreicht werden, die zusammen mit der Geometrie des Multikonnektors 60 einen Rastmechanismus ausbildet. Eine Verrastung kann jedoch auch an anderen Bauteilen erfolgen. Bei der in Fig. 3a dargestellten Situation handelt es sich um die Therapiestellung des Multikonnektors 60, für welche das an dem Multikonnektor 60 angebrachte Schlauchsegment (nicht dargestellt) automatisiert eingefädelt wurde. Zuvor wurde der Deckel 50 geschlossen, um beim Einfädelvorgang und während der Therapie den Eingriff in die Pumpe zu verhindern.

Der Deckel 50 weist einen Grundkörper auf, dessen Geometrie verschiedene Funktionsbereiche ausbildet. Beispielsweise weist der Deckel 50 auf der Außenseite eine Auswölbung 54 und eine Grifffläche 52 auf. Auf der gegenüber liegenden Seite dieser Grifffläche 52 ist an der Innenseite des Deckels 50 eine Anlagefläche 53 ausgeformt, an welcher der Multikonnektor 60 anliegt, wenn er in die Ausfädelposition geschwenkt wird. Dies ist in der Darstellung der Fig. 3b gezeigt. Das Gleiche gilt für die Druckfläche 55 im Bereich des Drehlagers, die in der Ausfädelposition an dem oberen Bereich des Multikonnektors 60 anliegt und so ein Herausfallen des Multikonnektors 60 aus der oberen Aufnahme 22 verhindert.

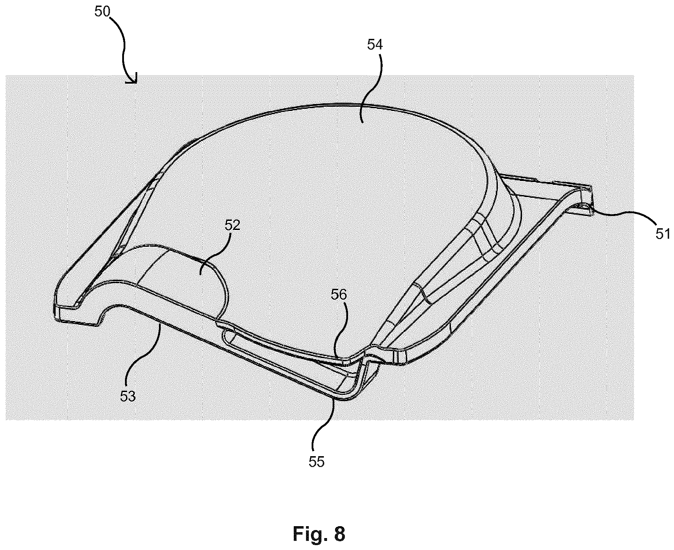

Dabei dient die Grifffläche 52 hauptsächlich als Gegenlager oder Bedien- und Betätigungsfläche zum Betätigen des jeweiligen Kippelements. Diese Grifffläche 52 kann zwar auch zum Öffnen des Deckels 50 verwendet werden, aber vorzugsweise ist die Geometrie des Deckels 50 im Bereich der Druckfläche 55 so ausgebildet, dass sich eine weitere Grifffläche ausbildet, mit welcher der Deckel 50 besser geöffnet werden kann. Diese Grifffläche 56 kann beispielsweise leicht nach außen gebogen sein, wie es der dreidimensionalen Ansicht der Fig. 8 zu entnehmen ist.

Um den unteren Teil des Multikonnektors 60 und somit das daran angebrachte Schlauchsegment 30 gegen den Deckel 50 zu schwenken, kann der Benutzer mit einer Hand die Grifffläche 52 am Deckel 50 und die Grifffläche 64 am Multikonnektor 60 greifen. Dabei kann er beispielsweise den Daumen auf die Grifffläche 52 des Deckels 50 legen und mit einem oder mehreren der anderen Finger unter die Grifffläche 64 am Multikonnektor 60 greifen, so dass er den Multikonnektor 60 mit diesen Fingern gegen den Deckel 50 ziehen kann, der dann ein Gegenlager bildet. Dieser Schwenkvorgang ist in der Fig. 3b durch einen Pfeil dargestellt.

Die Lage des Multikonnektors 60 gemäß Fig. 3b, in welcher der Multikonnektor 60 an der Innenseite des Deckels 50 anliegt, stellt für das daran angebrachte Schlauchsegment 30 die Ausfädelposition dar, in welcher einer oder mehrere Führungsstifte das Schlauchsegment 30 bei einer Drehung des Rotors 40 aus der Lauffläche herausheben können. Damit der Multikonnektor 60 in der Ausfädelposition gehalten werden kann, kann beispielsweise am oberen Drehlager ein Federmechanismus vorgesehen sein. Beim automatisierten Ausfädeln kann der Deckel 50 jedoch geschlossen bleiben, wobei sich das herausgehobene Schlauchsegment 30 nach dem Ausfädeln innerhalb der Auswölbung 54 des Deckels befindet. Anschließend kann der Deckel 50 geöffnet werden und der Multikonnektor 60 zusammen mit dem Schlauchsegment 30 aus der Pumpe entnommen werden, indem die Verrastung in der oberen Aufnahme 22 gelöst wird.

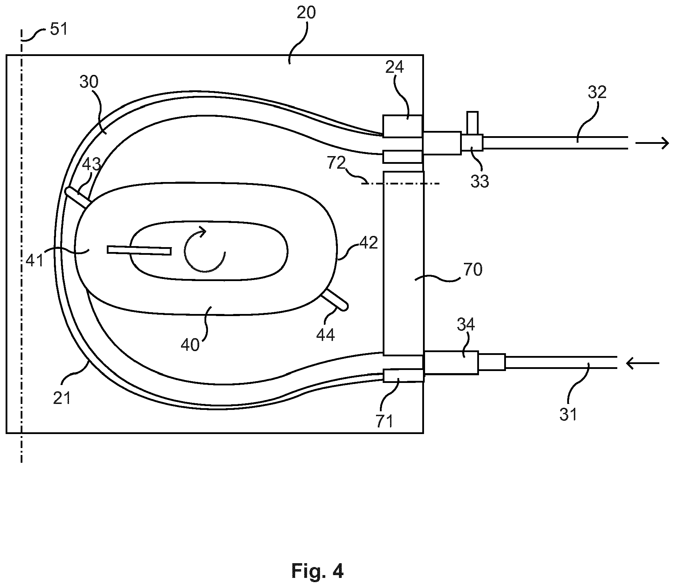

Fig. 4 stellt eine schematische Darstellung einer Schlauchrollenpumpe mit eingelegtem Schlauchsegment 30 und einer zweiten Ausführungsform eines Kippelements dar, das als Kipphebel 70 ausgebildet ist. Dieser Kipphebel 70 ist hierbei fester Bestandteil der Pumpe, aber der grundsätzliche Aufbau der Schlauchrollenpumpe entspricht dem bereits anhand der Fig. 2 beschriebenen Aufbau. Das Schlauchsegment 30 ist jedoch über einzelne Verbindungselemente 33 und 34 mit einer zuführenden Leitung 31 und einer abführenden Leitung 32 verbunden, wie es bei Standard-Überleitsystemen der Fall ist. Ein Ende des Schlauchsegments 30 ist in eine erste Aufnahme 24 eingebracht, die im oberen Bereich des Pumpengehäuses 20 ausgebildet ist. Diese Aufnahme 24 ist fest in das Pumpengehäuse 20 integriert und beispielsweise hohlzylindrisch mit einem Längsschlitz ausgeführt, so dass das Schlauchsegment 30 in diesen Längsschlitz eingedrückt werden kann.

Unterhalb der oberen (in Fig. 4) Aufnahme 24 ist der Kipphebel 70 angebracht, der um eine Schwenkachse 72 drehbar ist. Am schwenkbaren Ende des Kipphebels 70 ist eine zweite Aufnahme 71 ausgebildet, was ebenfalls durch einen Hohlzylinder mit Längsschnitt realisiert werden kann. In diesen Längsschnitt ist das andere Ende des Schlauchsegments 30 eingedrückt. Dabei liegen die Konnektoren 33 und 34 außen an den Aufnahmen 24 und 71 an, so dass die zu- und abführenden Leitungen 31, 32 nicht in die Pumpe hineingezogen werden können.

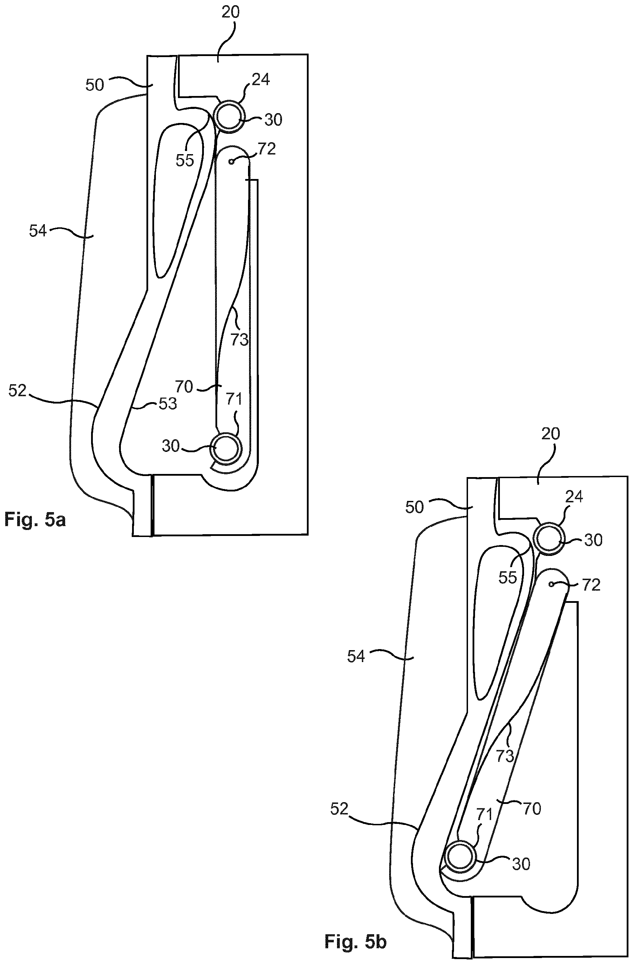

Die Figuren 5a und 5b zeigen den Kipphebel 70 analog zu den Figuren 3a und 3b in der Therapiestellung bzw. Einfädelposition und in der Ausfädelposition, in welcher das untere Ende des Schlauchsegments 30 soweit von dem Pumpenboden entfernt ist, dass ein Führungsstift am Rotor 40 das automatische Ausfädeln durchführen kann. Das Schlauchsegment 30 ist dabei in die obere Aufnahme 24 gedrückt, die sich fest am Pumpengehäuse befindet und das andere Ende des Schlauchsegments 30 ist in die untere Aufnahme 71 am Kipphebel 70 eingedrückt.

Auch an diesem Kipphebel 70 kann eine Grifffläche 73 ausgeformt sein, so dass der Kipphebel 70 über die Drehachse 72 in Richtung des geschlossenen Deckels 50 ziehbar ist. Dies kann wie bei der Ausführungsform mit Multikonnektor durch eine Hand erfolgen, wobei die Anlagefläche 53 des Deckels 50 erneut ein Gegenlager für den Kipphebel 70 in der Ausfädelposition bildet. In dieser Stellung der Fig. 5b kann die obere Druckfläche 55 des Deckels 50 an dem Schlauchsegment 30 anliegen, um ein Herausrutschen dieses Bereiches des Schlauchsegments 30 aus der Aufnahme 24 zu verhindern. Ferner kann im oberen Bereich des Kipphebels 70 ein weiterer Rastmechanismus vorgesehen sein, um den Kipphebel 70 in dieser Lage zu halten.

In beiden Ausführungsformen des Kippelements als montierbarer Multikonnektor 60 oder als fest angebrachter Kipphebel 70 können Detektionsmittel vorgesehen sein, um die Lage des jeweiligen Kippelements in der eingerasteten Einfädelposition direkt zu detektieren. Diese Einfädelposition stellt gleichzeitig die Therapieposition dar. Nach Einrasten des Kippelements in dieser Position wird der Deckel 50 geschlossen und der automatisierte Einfädelvorgang kann gestartet werden. Vorzugsweise sind ebenfalls Detektionsmittel zum Erkennen des Zustands des Deckels 50 vorgesehen, so dass der Einfädelvorgang gestartet werden kann, sobald über die Detektionsmittel sichergestellt ist, dass sich das Kippelement in der Einfädelposition befindet und der Deckel geschlossen ist.

Beim Einfädelvorgang wird der Rotor 40 langsam gedreht, so dass die Führungsstifte 41 und 42 den Einfädelvorgang durchführen. Dabei bewegt sich wenigstens ein Führungsstift außen entlang des Schlauchsegments 30 und drückt diesen zwischen den Rotor 40 und die Lauffläche 21. Um nach der Therapie den Ausfädelvorgang zu initiieren, wird das Kippelement von einem Benutzer zusammen mit dem Schlauchsegment 30 gegen den Deckel 50 gezogen. Dabei dient der Deckel 50 als Gegenlager, gegen den das Kippelement bis zum Anschlag gezogen werden kann. Dieser Anschlag definiert die Ausfädelposition, die ebenfalls durch Detektionsmittel erkannt werden kann. Bei geschlossenem Deckel 50 und dem jeweiligen Kippelement in der Ausfädelposition kann der Rotor 40 die Führungsstifte langsam zwischen den Pumpenboden und das Schlauchsegment 30 bewegen, wodurch das Schlauchsegment 30 aus dem Zwischenraum zwischen dem Rotor 40 und der Lauffläche 21 herausgehoben wird.

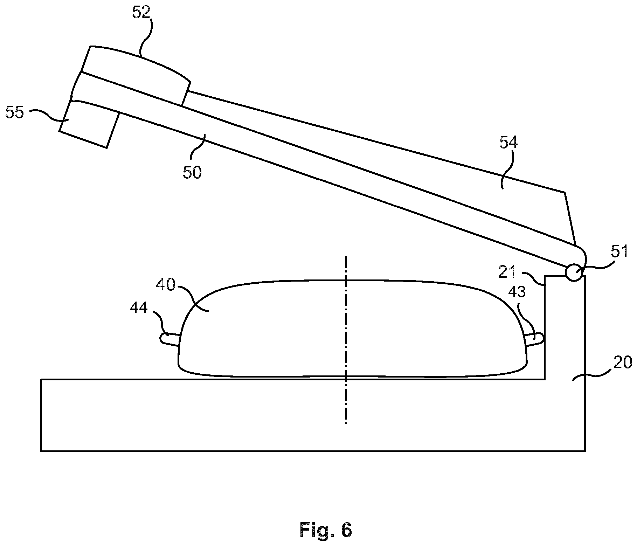

Fig. 6 zeigt einen schematischen Querschnitt durch eine Schlauchrollenpumpe mit leicht geöffnetem Deckel, wobei der Deckel 50 um die Drehachse 51 nach vorne geschwenkt ist und noch kein Schlauchsegment eingelegt wurde. Aus dieser Ansicht ist auch ersichtlich, dass die Auswölbung 54 an der Außenseite des Deckels 50 vorzugsweise in Richtung der Grifffläche 52 abflacht, da der Raum der Auswölbung 54 in der anderen Richtung am größten sein muss, um vor dem Einfädeln oder nach dem Ausfädeln das Schlauchsegment aufnehmen zu können.

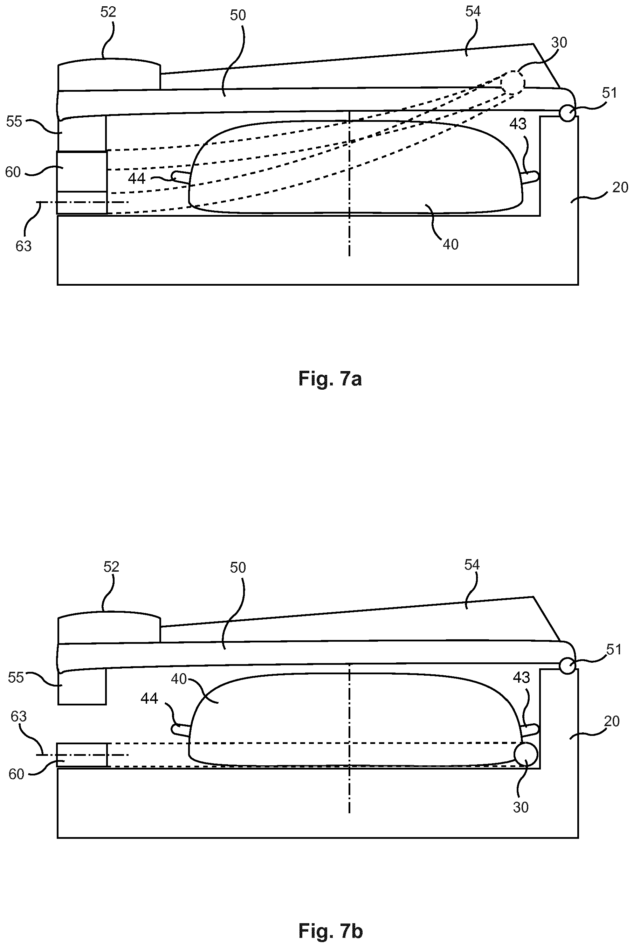

Fig. 7a zeigt den Querschnitt der Schlauchrollenpumpe mit geschlossenem Deckel 50, wobei das Schlauchsegment 30 beispielhaft an einem Multikonnektor 60 angebracht ist, der in der Ausfädelposition an dem Deckel 50 anliegt. Da es sich um eine Sicht von oben handelt, zeigt die schematische Darstellung der Fig. 7a auch, dass die obere Druckfläche 55 des Deckels 50 an dem oberen Konnektor des Multikonnektors 60 anliegt. Das Schlauchsegment 30 erstreckt sich innerhalb des Pumpengehäuses 20 und der Auswölbung 54 im Deckel 50, wobei der Verlauf des Schlauchsegments 30 gestrichelt dargestellt ist. Um das Schlauchsegment 30 in diese Lage heben zu können, hat sich beim Drehen des Rotors 40 wenigstens ein Führungsstift43, 44 zwischen Schlauchsegment 30 und dem Boden des Pumpengehäuses 20 bewegt.

Fig. 7b zeigt den Querschnitt der Schlauchrollenpumpe mit geschlossenem Deckel 50, wobei der Multikonnektor 60 in die Einfädelposition gebracht wurde und das Schlauchsegment 30 bereits zwischen dem Rotor 40 und der Lauffläche 21 eingefädelt ist. Um das Schlauchsegment 30 in diese Lage einfädeln zu können, hat sich beim Drehen des Rotors 40 wenigstens ein Führungsstift 43, 44 zwischen dem Schlauchsegment 30 und dem Deckel 50 bewegt.

Darüber hinaus zeigt die Fig. 8 eine dreidimensionale Ansicht des Deckels 50 mit der hohlen Auswölbung 54 und der äußeren Grifffläche 52. Auf der gegenüber liegenden Seite der Grifffläche 52 ist die Anlagefläche 53 ausgebildet und die Druckfläche 55 wird durch eine hohle Verdickung in diesem Bereich geformt. Im Bereich der Druckfläche 55 bildet der Deckel auf der gegenüber liegenden Seite die Grifffläche 56 zum Öffnen des Deckels 50 aus. Ferner ist ersichtlich, wie die Auswölbung 54 in Richtung der Grifffläche 52 abflacht.

### Bezugszeichenliste

- 10: Medizinisches Gerät zur extrakorporalen Blutbehandlung, Dialysegerät
- 11: Arterieller Luftfänger
- 12: Venöser Luftfänger
- 13: Dialysator
- 14: Dialysat
- 15: Anzeige-/Eingabeeinheit, Touchscreen
- 16: Steuer-/Regelungseinheit
- 20: Pumpengehäuse
- 21: Lauffläche
- 22: Aufnahme, Drehlager, Rastgeometrie
- 23,24: Aufnahme, Rastgeometrie
- 30: Pumpensegment, Schlauchsegment
- 31: Leitung, zuführend, arterielles Blut vom Patienten
- 32: Leitung, abführend, Venöses Blut zum Patienten
- 33,34: Konnektor, Verbindungselement
- 40: Rotor
- 41,42: Hauptscheitel
- 43,44: Führungsstift
- 50: Deckel, Pumpendeckel
- 51: Scharnier, Drehachse des Deckels
- 52: Grifffläche
- 53: Anlagefläche
- 54: Auswölbung
- 55: Druckfläche
- 56: Grifffläche
- 60: Kippelement, Multikonnektor
- 61,62: Konnektor, Konnektorelement
- 63: Drehachse des Multikonnektors
- 64: Grifffläche des Multikonnektors
- 70: Kippelement, Kipphebel
- 71: Aufnahme
- 72: Drehachse des Kipphebels
- 73: Grifffläche des Kipphebels

## Patentansprüche

1. Schlauchrollenpumpe für ein medizinisches Gerät (10) zur extrakorporalen Blutbehandlung, umfassend ein Pumpengehäuse (20), das eine gebogene Lauffläche (21) und einen innerhalb der gebogenen Lauffläche (21) drehbaren Rotor (40) aufweist, wobei ein Schlauchsegment (30) schlaufenförmig zwischen die Lauffläche (21) und den Rotor (40) einbringbar ist und am Pumpengehäuse (20) Mittel (60; 70) zur Anbringung des Schlauchsegments (30) vorgesehen sind, mit denen wenigstens ein Ende des Schlauchsegments (30) um eine Drehachse (63; 72) schwenkbar am Pumpengehäuse (20) anbringbar ist, wobei das Verschwenken des Schlauchsegments (30) durch Verschwenken eines Kippelements (60; 70) durchführbar ist, an dem das Schlauchsegment (30) wenigstens teilweise angebracht ist,
**dadurch gekennzeichnet, dass**
ein Deckel (50) schwenkbar am Pumpengehäuse (20) gelagert ist, mit dem das Pumpengehäuse (20) wenigstens teilweise abdeckbar ist, wobei der Deckel (50) eine einen Schwenkraum definierende Ausformung aufweist und einen Anschlag ausbildet, so dass er im geschlossenen Zustand bei Verschwenken des Schlauchsegments (30) in Richtung des geschlossenen Deckels (50) ein Gegenlager für das Kippelement (60; 70) bildet, durch welches das daran angebrachte Schlauchsegment (30) in einer definierten, verschwenkten Lage gegenüber dem Pumpengehäuse (20) ausrichtbar ist, und
die Schlauchrollenpumpe wenigstens Mittel (43, 44) zum automatisierten Ausfädeln des Schlauchsegments (30) aus der gebogenen Lauffläche (21) in dieser definierten, verschwenkten Lage aufweist.

2. Schlauchrollenpumpe nach Anspruch 1,
**dadurch gekennzeichnet, dass** das Kippelement (70) fester Bestandteil der Schlauchrollenpumpe ist und Mittel (71) zur lösbaren Anbringung wenigstens eines Endes des Schlauchsegments (30) aufweist.

3. Schlauchrollenpumpe nach Anspruch 2,
**dadurch gekennzeichnet, dass** das Kippelement (70) Mittel (71) zur lösbaren Anbringung eines Endes des Schlauchsegments (30) aufweist, während am Pumpengehäuse (20) Mittel (24) zur lösbaren Anbringung des anderen Endes des Schlauchsegments (30) vorgesehen sind.

4. Schlauchrollenpumpe nach Anspruch 1,
**dadurch gekennzeichnet, dass** das Kippelement (60) lösbar und schwenkbar an dem Pumpengehäuse (20) anbringbar ist, indem es in eine Aufnahme (22) am Pumpengehäuse (20) einbringbar ist, welche ein Drehlager für das Kippelement (60) bildet, wobei das Drehlager im Bereich eines Endes des Schlauchsegments (30) angeordnet ist.

5. Schlauchrollenpumpe nach einem oder mehreren der Ansprüche 1 bis 4, **dadurch gekennzeichnet, dass** der Deckel (50) an seiner Innenseite, welche im geschlossenen Zustand dem Kippelement (60; 70) zugewandt ist, eine Druckfläche (55) aufweist, die im Bereich des Drehlagers an dem Kippelement (60; 70) und/oder dem Schlauchsegment (30) anliegt.

6. Schlauchrollenpumpe nach einem oder mehreren der Ansprüche 1 bis 5, **dadurch gekennzeichnet, dass** das Kippelement (60; 70) um eine Drehachse (63; 72) schwenkbar am Pumpengehäuse (20) angebracht ist, die quer zur Drehachse (51) des Deckels (50) am Pumpengehäuse (20) verläuft.

7. Schlauchrollenpumpe nach einem oder mehreren der Ansprüche 1 bis 6, **dadurch gekennzeichnet, dass** das Kippelement (60; 70) bei geschlossenem Deckel (50) wenigstens teilweise von außen zugänglich ist, insbesondere von einer Seite zugänglich ist, die gegenüber der Drehachse (51) des Deckels (50) liegt.

8. Schlauchrollenpumpe nach Anspruch 7,
**dadurch gekennzeichnet, dass** die Geometrie des Kippelements (60; 70) eine Grifffläche (64; 73) ausbildet, mit welcher das Kippelement (60; 70) gegen den geschlossenen Deckel (50) ziehbar ist.

9. Schlauchrollenpumpe nach Anspruch 8,
**dadurch gekennzeichnet, dass** die Geometrie des Deckels (50) an seiner Außenseite, welche im geschlossenen Zustand von dem Kippelement (60;70) weg weist, eine Grifffläche (52) ausbildet, die bei Kontakt des Kippelements (60;70) mit dem Deckel (50) als Gegenlager in einem Abstand zu der Grifffläche (52;73) des Kippelements (60;70) steht, der größer als 3 mm ist, insbesondere in der Größenordnung von 5 mm bis 20 mm liegt.

10. Schlauchrollenpumpe nach einem oder mehreren der Ansprüche 1 bis 9, **dadurch gekennzeichnet, dass** die Geometrie des Kippelements (60; 70) und die Geometrie des Deckels (50) so aufeinander abgestimmt sind, dass das Kippelement (60; 70) nach dem Verschwenken gegen den geschlossenen Deckel (50) als Gegenlager flächig an einer Anlagefläche (53) des Deckels (50) anliegt.

11. Schlauchrollenpumpe nach einem oder mehreren der Ansprüche 1 bis 10, **dadurch gekennzeichnet, dass** der Deckel (50) bereichsweise eine hohle Auswölbung (54) in eine Richtung aufweist, die im geschlossenen Zustand des Deckels (50) von dem Pumpengehäuse (20) weg weist.

12. Schlauchrollenpumpe nach Anspruch 11,
**dadurch gekennzeichnet, dass** die Seitenkontur der hohlen Auswölbung (54) im geschlossenen Zustand des Deckels (50) im Wesentlichen der Kontur der Lauffläche (21) folgt.

13. Schlauchrollenpumpe nach einem oder beiden der Ansprüche11 und 12, **dadurch gekennzeichnet, dass** die hohle Auswölbung (54) im geschlossenen Zustand des Deckels (50) in Richtung des Kippelements (60; 70) abflacht.

14. Medizinisches Gerät zur extrakorporalen Blutbehandlung, umfassend wenigstens eine Schlauchrollenpumpe mit einem Pumpengehäuse (20), das eine gebogene Lauffläche (21) und einen innerhalb der Lauffläche (21) drehenden Rotor (40) aufweist, wobei ein Schlauchsegment (30) eines extrakorporalen Blutkreislaufs zwischen die Lauffläche (21) und den Rotor (40) einbringbar ist, und dem Schlauchsegment (30) Blut über eine zuführende Leitung (31) zuführbar ist, während Blut von dem Schlauchsegment (30) über eine abführende Leitung (32) abführbar ist, **dadurch gekennzeichnet, dass** das Gerät eine Schlauchrollenpumpe gemäß einem oder mehreren der Ansprüche 1 bis 13 umfasst.

## Claims

1. A tube roller pump for a medical device (10) for extracorporeal blood treatment comprising a pump housing (20) having a bent bearing surface (21) and a rotor (40) rotatable inside the bent bearing surface (21), wherein a tubing segment (30) can be introduced between the bearing surface (21) and the rotor (40) in loop shape and at the pump housing (20) means (60; 70) for arranging the tubing segment (30) are provided by which at least one end of the tubing segment (30) can be arranged at the pump housing (20) so as to be pivotable about an axis of rotation (63, 72), wherein the tubing segment (30) can be pivoted by pivoting a tilting element (60; 70) on which the tubing segment (30) is at least partly mounted,
**characterized in that**
a cover (50) is pivotably arranged on the pump housing (20) and can at least partly cover the pump housing (20), the cover (50) comprises a molding defining a pivoting space and forms a stop so that in the closed state upon pivoting the tubing segment (30) in the direction of the closed cover (50) it constitutes a counter-bearing for the tilting element (60; 70) by which the tubing segment (30) mounted thereon can be aligned at a defined, pivoted position relative the pump housing (20), and
the tube roller pump includes at least means (43, 44) for automated unthreading of the tubing segment (30) from the bent bearing surface (21) at said defined, pivoted position.

2. The tube roller pump according to claim 1, **characterized in that** the tilting element (70) is a fixed component of the tube roller pump and includes means (71) for detachably arranging at least one end of the tubing segment (30).

3. The tube roller pump according to claim 2, **characterized in that** the tilting element (70) includes means (71) for detachably mounting one end of the tubing segment (30), whereas means (24) for detachably mounting the other end of the tubing segment (30) are provided at the pump housing (20).

4. The tube roller pump according to claim 1, **characterized in that** the tilting element (60) is adapted to be detachably and movably mounted on the pump housing (20) by being adapted to be introduced into a receiving portion (22) at the pump housing (20) which forms a pivot bearing for the tilting element (60), the pivot bearing being arranged in the area of one end of the tubing segment (30).

5. The tube roller pump according to one or more of claims 1 to 4, **characterized in that** the cover (50) includes at its inside, which faces the tilting element (60; 70) in the closed state, a contact face (55) which contacts the tilting element (60; 70) and/or the tubing segment (30) in the area of the pivot bearing.

6. The tube roller pump according to one or more of claims 1 to 5, **characterized in that** the tilting element (60; 70) is arranged at the pump housing (20) so as to be pivotable about an axis of rotation (63; 72) extending transversely to the axis of rotation (51) of the cover (50) at the pump housing (20).

7. The tube roller pump according to one or more of claims 1 to 6, **characterized in that,** when the cover (50) is closed, the tilting element (60; 70) is accessible at least partly from outside, in particular from a side which lies opposite from the axis of rotation (51) of the cover (50).

8. The tube roller pump according to claim 7, **characterized in that** the geometry of the tilting element (60; 70) forms a gripping surface (64; 73) by which the tilting element (60; 70) can be pulled against the closed cover (50).

9. The tube roller pump according to claim 8, **characterized in that** at its outside facing away from the tilting element (60; 70) in the closed state the geometry of the cover (50) forms a gripping surface (52) which when the tilting element (60; 70) contacts with the cover (50) as a counter-bearing is at a distance from the gripping surface (52; 73) of the tilting element (60; 70) which is larger than 3 mm, in particular in the size range of 5 mm to 20 mm.

10. The tube roller pump according to one or more of claims 1 to 9, **characterized in that** the geometry of the tilting element (60; 70) and the geometry of the cover (50) are adjusted to each other so that, after pivoting against the closed cover (50) as a counter-bearing, the tilting element (60; 70) is adjacent with its full surface to a mating surface (53) of the cover (50).

11. The tube roller pump according to one or more of claims 1 to 10, **characterized in that** the cover (50) in portions has a hollow bulge (54) in a direction facing away from the pump housing (20) in the closed state of the cover (50).

12. The tube roller pump according to claim 11, **characterized in that** the side contour of the hollow bulge (54) in the closed state of the cover (50) substantially follows the contour of the bearing surface (21).

13. The tube roller pump according to one or both of claims 11 and 12, **characterized in that** the hollow bulge (54) in the closed state of the cover (50) flattens in the direction of the tilting element (60; 70).

14. A medical device for extracorporeal blood treatment comprising at least a tube roller pump including a pump housing (20) having a bent bearing surface (21) and a rotor (40) rotating inside the bearing surface (21), wherein a tubing segment (30) of an extracorporeal blood circulation can be introduced between the bearing surface (21) and the rotor (40) and blood can be fed to the tubing segment (30) via a feeding line (31), while blood can be discharged from the tubing segment (30) via a discharging line (32), **characterized in that**
the device comprises a tube roller pump according to one or more of claims 1 to 13.

## Revendications

1. Pompe à rouleau tubulaire pour un appareil (10) médical servant au traitement extracorporel du sang, comprenant un corps de pompe (20), qui présente une surface de roulement (21) pliée et un rotor (40) pouvant tourner à l'intérieur de la surface de roulement (21) pliée, dans laquelle un segment tubulaire (30) peut être introduit de manière à présenter une forme de boucle entre la surface de roulement (21) et le rotor (40) et des moyens (60 ; 70) servant à installer le segment tubulaire (30) sont prévus au niveau du corps de pompe (20), à l'aide desquels au moins une extrémité du segment tubulaire (30) peut être installée au niveau du corps de pompe (20) de manière à pouvoir pivoter autour d'un axe de rotation (63 ; 72), dans laquelle le pivotement du segment tubulaire (30) peut être effectué par le pivotement d'un élément basculant (60 ; 70), au niveau duquel le segment tubulaire (30) est installé au moins en partie,
**caractérisée en ce que**
un couvercle (50) est monté de manière à pouvoir pivoter au niveau du corps de pompe (20), par lequel le corps de pompe (20) peut être recouvert au moins en partie, dans laquelle le couvercle (50) présente une déformation définissant un espace de pivotement et réalise une butée, de sorte qu'il forme, dans l'état fermé, lors du pivotement du segment tubulaire (30) en direction du couvercle (50) fermé, un contre-palier pour l'élément basculant (60 ; 70), par lequel le segment tubulaire (30) installé au niveau de ce dernier peut être orienté dans une position pivotée définie par rapport au corps de pompe (20), et
la pompe à rouleau tubulaire présente au moins des moyens (43, 44) servant à retirer de manière automatique le segment tubulaire (30) hors de la surface de roulement (21) pliée dans ladite position pivotée définie.

2. Pompe à rouleau tubulaire selon la revendication 1,
**caractérisée en ce que** l'élément basculant (70) est un composant fixe de la pompe à rouleau tubulaire et présente des moyens (71) servant à installer de manière amovible au moins une extrémité du segment tubulaire (30).

3. Pompe à rouleau tubulaire selon la revendication 2,
**caractérisée en ce que** l'élément basculant (70) présente des moyens (71) servant à installer de manière amovible une extrémité du segment tubulaire (30), tandis que des moyens (24) servant à installer de manière amovible l'autre extrémité du segment tubulaire (30) sont prévus au niveau du corps de pompe (20).

4. Pompe à rouleau tubulaire selon la revendication 1,
**caractérisée en ce que** l'élément basculant (60) peut être installé de manière amovible et de manière à pouvoir pivoter au niveau du corps de pompe (20) **en ce qu'**il peut être introduit dans un logement (22) au niveau du corps de pompe (20), qui forme un palier rotatif pour l'élément basculant (60), dans laquelle le palier rotatif est disposé dans la zone d'une extrémité du segment tubulaire (30).

5. Pompe à rouleau tubulaire selon l'une quelconque ou plusieurs des revendications 1 à 4,
**caractérisée en ce que** le couvercle (50) présente au niveau de con côté intérieur, qui est tourné, dans l'état fermé, vers l'élément basculant (60 ; 70), une surface de pression (55), qui repose, dans la zone du palier rotatif, au niveau de l'élément basculant (60 ; 70) et/ou au niveau du segment tubulaire (30).

6. Pompe à rouleau tubulaire selon l'une quelconque ou plusieurs des revendications 1 à 5,
**caractérisée en ce que** l'élément basculant (60 ; 70) est installé au niveau du corps de pompe (20) de manière à pouvoir pivoter autour d'un axe de rotation (63 ; 72), qui s'étend de manière transversale par rapport à l'axe de rotation (51) du couvercle (50) au niveau du corps de pompe (20).

7. Pompe à rouleau tubulaire selon l'une quelconque ou plusieurs des revendications 1 à 6,
**caractérisée en ce que** l'élément basculant (60 ; 70) est accessible, lorsque le couvercle (50) est fermé, au moins en partie depuis l'extérieur, en particulier est accessible depuis un côté, qui se trouve en vis-à-vis de l'axe de rotation (51) du couvercle (50).

8. Pompe à rouleau tubulaire selon la revendication 7,
**caractérisée en ce que** la géométrie de l'élément basculant (60 ; 70) réalise une surface de préhension (64 ; 73), par laquelle l'élément basculant (60 ; 70) peut être tiré contre le couvercle (50) fermé.

9. Pompe à rouleau tubulaire selon la revendication 8,
**caractérisée en ce que** la géométrie du couvercle (50) réalise, au niveau de son côté extérieur, qui pointe, dans l'état fermé, de manière à s'éloigner de l'élément basculant (60 ; 70), une surface de préhension (52), qui se trouve, en cas de contact de l'élément basculant (60 ; 70) avec le couvercle (50), en tant que contre-palier, à une distance par rapport à la surface de préhension (52 ; 73) de l'élément basculant (60 ; 70), laquelle distance est supérieure à 3 mm, en particulier est de l'ordre de 5 mm à 20 mm.

10. Pompe à rouleau tubulaire selon l'une quelconque ou plusieurs des revendications 1 à 9,
**caractérisée en ce que** la géométrie de l'élément basculant (60 ; 70) et la géométrie du couvercle (50) sont adaptées l'une à l'autre de telle sorte que l'élément basculant (60 ; 70) repose après le pivotement contre le couvercle (50) fermé en tant que contre-palier à plat au niveau d'une surface d'appui (53) du couvercle (50).

11. Pompe à rouleau tubulaire selon l'une quelconque ou plusieurs des revendications 1 à 10,
**caractérisée en ce que** le couvercle (50) présente par endroits un bombement (54) creux dans une direction, qui pointe, dans l'état fermé du couvercle (50) de manière à s'éloigner du corps de pompe (20).

12. Pompe à rouleau tubulaire selon la revendication 11,
**caractérisée en ce que** le contour latéral du bombement (54) creux suit, dans l'état fermé du couvercle (50), sensiblement le contour de la surface de roulement (21).

13. Pompe à rouleau tubulaire selon l'une quelconque ou les deux des revendications 11 et 12,
**caractérisée en ce que** le bombement (54) creux s'aplatit, dans l'état fermé du couvercle (50), en direction de l'élément basculant (60 ; 70).

14. Appareil médical servant au traitement extracorporel du sang, comprenant au moins une pompe à rouleau tubulaire avec un corps de pompe (20), qui présente une surface de roulement (21) pliée et un rotor (40) tournant à l'intérieur de la surface de roulement (21), dans lequel un segment tubulaire (30) d'une circulation extracorporelle de sang peut être introduit entre la surface de roulement (21) et le rotor (40) et du sang peut être amené au segment tubulaire (30) par l'intermédiaire d'un conduit d'arrivée (31), tandis que du sang peut être évacué du segment tubulaire (30) par l'intermédiaire d'un conduit d'évacuation (32),
**caractérisé en ce que** l'appareil comprend une pompe à rouleau tubulaire selon l'une quelconque des revendications 1 à 13.
